# EUROPEAN PATENT SPECIFICATION

(11) **EP 1 681 060 B1**
(45) Date of publication and mention of the grant of the patent: **19.05.2010**
(21) Application number: 05000776.4
(22) Date of filing: 15.01.2005
(51) Int. Cl.: A61K 36/02, A61K 35/74, C12R 1/01, C12P 23/00, C12R 1/89

(54) **Improved process for obtaining carotene and carotenoids from algae or cyanobacteria**
Verbessertes Verfahren zur Herstellung von Karotene und Karotenoiden aus Algen und Cyanobakterien
Procédé amélioré pour produire des carotenes et des carotenoides à partir des algues et des Cyanobactéries

(43) Date of publication of application: 19.07.2006
(73) Proprietor: Cognis IP Management GmbH, 40589 Düsseldorf (DE)
(72) Inventor: Johannisbauer, Wilhelm, Dr., 40699 Erkrath (DE); Mahnke, Eike Ulf, Dr., 40878 Ratingen (DE); Sander, Andreas, Dr., 89257 Illertissen (DE); Weiss, Albrecht, Dr., 40764 Langenfeld (DE); Pulz. Otto, Dr. Dr. h.c., 14558 Bergholz-Rehbrücke (DE); Franke, Horst, 14558 Bergholz-Rehbrücke (DE)
(74) Representative: Reinhardt, Jürgen

(56) References cited:
- WO-A-97/28274
- MA RAYMOND YIN-NIN ET AL: "Induction of astaxanthin formation by reactive oxygen species in mixotrophic culture of Chlorococcum sp" BIOTECHNOLOGY LETTERS, vol. 23, no. 7, April 2001 (2001-04), pages 519-523, XP008049541 ISSN: 0141-5492
- HARKER MARK ET AL: "Autotrophic growth and carotenoid production of Haematococcus pluvialis in a 30 liter air-lift photobioreactor" JOURNAL OF FERMENTATION AND BIOENGINEERING, vol. 82, no. 2, 1996, pages 113-118, XP002334962 ISSN: 0922-338X
- LEON R ET AL: "Microalgae mediated photoproduction of beta-carotene in aqueous-organic two phase systems" BIOMOLECULAR ENGINEERING, ELSEVIER, NEW YORK, NY, US, vol. 20, no. 4-6, July 2003 (2003-07), pages 177-182, XP004446910 ISSN: 1389-0344
- DATABASE WPI Section Ch, Week 199516 Derwent Publications Ltd., London, GB; Class D13, AN 1995-117865 XP002031373 -& JP 07 039389 A (HIGASHIMARU SHOYU KK) 10 February 1995 (1995-02-10)
- BOUSSIBA ET AL: "Astaxanthin accumulation in the green alga Haematococcus pluvialis" CHEMICAL ABSTRACTS + INDEXES, AMERICAN CHEMICAL SOCIETY. COLUMBUS, US, vol. 25, no. 115, 23 December 1991 (1991-12-23), XP002031370 ISSN: 0009-2258
- BOUSSIBA S: "Carotenogenesis in the green alga Haematococcus pluvialis: Cellular physiology and stress response" PHYSIOLOGIA PLANTARUM, vol. 108, no. 2, February 2000 (2000-02), pages 111-117, XP008049523 ISSN: 0031-9317
- HARKER MARK ET AL: "Factors responsible for astaxanthin formation in the chlorophyte Haematococcus pluvialis" BIORESOURCE TECHNOLOGY, vol. 55, no. 3, 1996, pages 207-214, XP002334963 ISSN: 0960-8524
- KOBAYASHI MAIKO ET AL: 'Enhanced carotenoid biosynthesis by oxidative stress in acetate-induced cyst cells of a green unicellular alga, Haematococcus pluvialis Kobayashi' APPLIED AND ENVIRONMENTAL MICROBIOLOGY vol. 59, no. 3, 1993, pages 867 - 873
- DOMÍNGUEZ-BOCANEGRA A.R. ET AL: 'Influence of environmental and nutritional factors in the production of astaxanthin from Haematococcus pluvialis' BIORESOURCE TECHNOLOGY vol. 92, no. 2, 2004, pages 209 - 214
- ZHANG X.W. ET AL: 'Kinetic models for astaxanthin production by high cell density mixotrophic culture of the microalga Haematococcus pluvialis' JOURNAL OF INDUSTRIAL MICROBIOLOGY AND BIOTECHNOLOGY vol. 23, no. 1, 1999, pages 691 - 696
- PELAH D. ET AL: 'The effect of salt stress on the production of canthaxanthin and astaxanthin by Chlorella zofingiensis grown under limited light intensity' WORLD JOURNAL OF MICROBIOLOGY & BIOTECHNOLOGY vol. 20, no. 5, 2004, pages 483 - 486
- KOBAYASHI M. ET AL: 'Light-independent, astaxanthin production by the green microalga Haematococcus pluvialis under salt stress' BIOTECHNOLOGY LETTERS vol. 19, no. 6, 1997, pages 507 - 509

## Description

### Object of the invention

The present invention relates to an improved process for obtaining carotenes or carotenoids from algae or cyanobacteria, especially for the manufacture of astaxanthin from *Haematococcus* strains.

### State of the art

The cultivation of algae, particularly *Haematococcus pl*. for the production of carotenes and carotenoids, for example, beta-carotene or astaxanthin is divided in a so called "Green Phase" that is focused on the growth of the algae mass and a "Red Phase" for the production of the carotenoids in the cells. Both parts of the cultivation process have to be optimised to get as much carotenoid as possible per time unit in a given photo bioreactor volume. An overview is given in: Amos Richmond, "Handbook of micro-algae culture: Biotechnology and Applied Physiology", pp 70-72 (2004), Blackwell Science Ltd, Oxford**,** where also the negative influence of high oxygen concentrations in the cultivation of the algae *Spirulina* is discussed and a photo-inhibition for the production is stated.

EP 0877817 B1 (Ben Gurion University) is related to a procedure for large-scale production of astaxanthin from *Haematococcus.* In more detail, the patent teaches a two-phase cultivation process, the first phase provides for the maintenance of optimal vegetative growth of the cells under controlled environmental conditions. This first stage or phase is characterized by the cells having continuous cell division and an astaxanthin/chlorophyll ratio between about 0.1 to 0.4, and an increase in chlorophyll content during the cultivation process. In the second phase of this procedure, induction of astaxanthin production and accumulation within the cells was achieved by collecting the cells from the above green cell culture and subjecting them to stress by nutrient deprivation by re-suspending the cells in tap water enriched with CO₂, followed by cultivation of the cells under exposure to high light intensities, e.g., full sunlight.

Boussiba describes the carotenogenesis in the green algae *Haematococcus pluvialis* **[**Physiol. Plant 108: 111-117 (2000**)]** and has studied the various environmental stress conditions, as light intensities, nitrogen and phosphate limitations, and salt stress. He suggests that beside high light intensities, reactive oxygen species, e. g., oxygen radicals generated through addition of photo sensing dyes to the culture suspension, mediate the induction of astaxanthin synthesis.

Reference is also made to Biotechnology Letters, Vol. 23(7), p. 519-523 (2001**).** This document discloses a process according to which the micro-algae *Chlorococcum sp.* is subjected to chemical stress by addition of hydrogen peroxide in order to improve stimulation of astaxanthin production. The stressing, however, takes placing during the so-called "Green Phase".

The production costs of carotenes and carotenoids, e.g. beta-carotene, lycopene, lutein, and especially astaxanthin from algae are high because of the large reactor volumes and the high light intensities for each algae cell in the so-called Red Phase. Usually the light is not able to penetrate deeper than about 5 cm into high density algae fermentations. These optimal light conditions for the Red Phase are not available in Northern Europe.

Therefore, the objective which underlies the present invention has been to develop a process to produce carotenoids, particularly astaxanthin, in the Red Phase at about the same low light intensities as in the Green Phase in order to economically produce carotenoids also under European light conditions. Therefore, more particularly, the main aims have been
● Separation of the cultivation into two main steps, that each step may be carried out in separate reactors using different culture media,
● Controlled addition of C-containing nutrients in the growth phase (Green Phase),
● Controlled process of cell dividing and cell enlarging (Green Phase),
● Induction of astaxanthin synthesis under the same low light conditions after reduction of nutrients and fertilizers when switching to the Red Phase.

### Description of the invention

The present invention claims an improved process for the production of carotenes or carotenoids from algae or cyanobacteria comprising
(a) cultivating algae or cyanobacteria having the ability for the biosynthesis of carotenes or carotenoids under conditions suitable for optimal vegetable growth of said cells in the so-called "Green Phase", and
(b) collecting the cells thus obtained and cultivating them [at temperatures in the range of 5 to 35 °C and light intensities of 30 to 200 µE*m⁻²*s⁻¹ in the so-called "Red Phase",
which is characterised in that the light intensity in step (b) is not increased compared to step (a) and that the cultivated cells are subjected to chemical stress by adding a substance releasing active oxygen species for the induction of the carotene or carotenoid synthesis in one step or repeatedly over the whole period of the Red Phase.

In order to solve the problem described above, applicant has carried out systematic research in order to optimise the reaction conditions in the Green as well as in the Red Phases. The growth phase has been established as mixotrophic cultivation with the controlled addition of nutrients like glucose or sodium acetate besides the use of carbon dioxide in order to assure fast cell growth. By this method the growth rate or the productivity for the algae mass was improved by a factor of 5 to 10. For further improvements of the growth phase it has been found advantageous to have two stages with
(i) controlled cell division and
(ii) enlarging the small cells before switching to the Red Phase (carotenoids production).

Different stress factors have also been tested to induce the carotenoid formation in the Red Phase. The algae are preparing to survive, and a generally discussed theory is that the carotenoids are produced to protect the photo-system of the algae. Therefore, a common approach from the state of the art has been to start the Red Phase with a much higher light intensity than compared to the Green Phase.

Surprisingly, applicant has found that an even higher production level of carotenoids in general, and more particularly of astaxanthin, can be achieved maintaining the same light conditions over the whole fermentation (Green and Red Phases) if the suspension of algae is subjected to chemical stress by adding substances releasing active oxygen species, particularly by addition of peroxides, and more particularly hydrogen peroxide. According to the process of the invention, an enhanced induction of the carotenoid pathway is achieved, which leads to a higher level of enzymes directly involved in metabolism. This goes along with an enhanced biosynthesis of carotenoids in general, and more particularly of astaxanthin.

The best results achieved by addition of hydrogen peroxide resulted in an economically important reduction of cultivation time by a factor of 5 to 8 to achieve the same astaxanthin concentrations compared to a high light intensity induction with the same strain. For example, in total the manufacturing costs of algae containing 3 to 4 % b.w. astaxanthin could be reduced by a factor of 5 to 10 against the state of the art known from literature.

### Carotenes and Carotenoids

Carotenes represent a group of unsaturated tetraterpenes having 11 to 12 double bonds. Of major importance are the three isomeric α-, β- und γ-carotenes, which all show the same structure comprising 9 conjugated double bonds and a β-ionon moiety at the end of the molecule. In the following, a couple of carotenes are shown which can be obtained from algae:

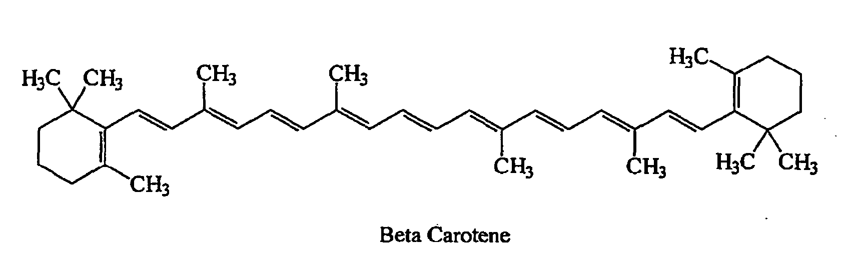

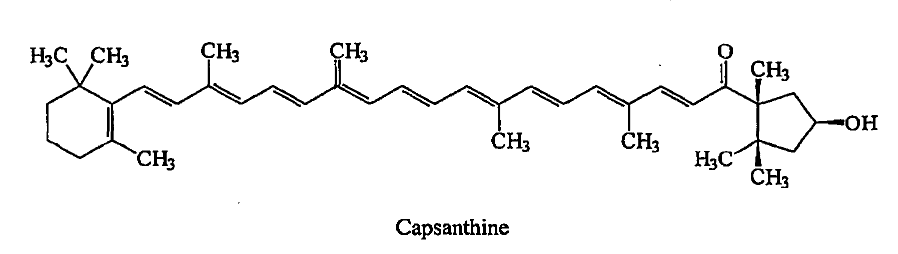

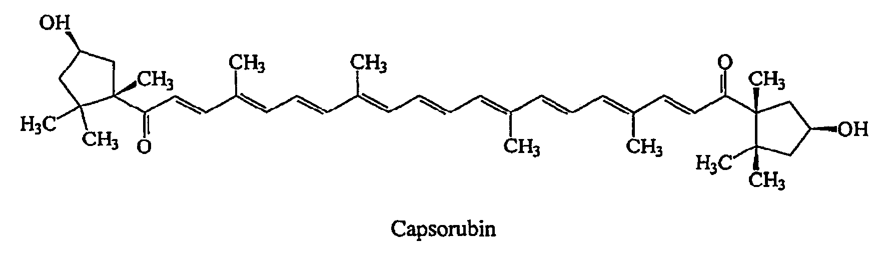

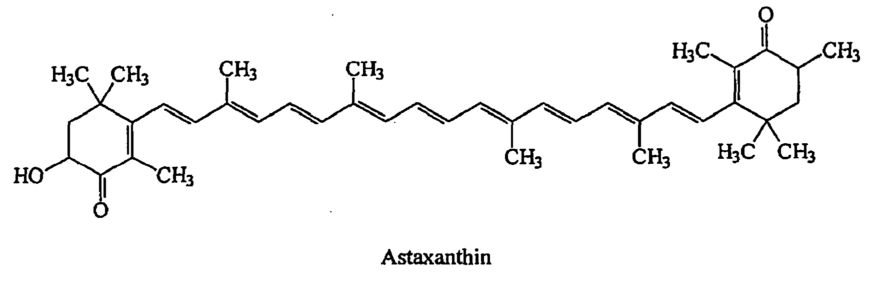

Beside the isomers already mentioned, one can also take into account δ-, ε- and ζ-carotene (lycopene), although β-carotene (pro-vitamin A) is of major importance, which can be split into two molecules of retinal by certain enzymes. Oxygen-containing derivatives of said carotenes are called carotenoids or xanthophylls. Their general structure consists of 8 isoprene units (tetraterpenes). One can consider carotenoids to be formed by two C₂₀-isoprenoids which are linked in a way that the central methyl groups are in 1,6 position. Typical examples are (3R,6'R)-β-ε-Carotene-3,3'-diol (lutein), (3R,3'S,5'R)-3,3'-Dihydroxy-β,κ-carotene-6-on (capsanthine), 9'-cis-6,6'-diapocarotene dicarboxylic acid-6'-methyl ester (bixin), (3S,3'S,5R,5'R)-3,3'-Dihydroxy-κ,κ-carotene-6,6'-dion (capsorubin) and especially (3S,3'S)-3,3'-Dihydroxy-β,β'-carotene-4,4'-dion (astaxanthin). Further carotenoids of the present invention are selected from the group consisting of lutein, lycopene, violaxanthin, cryptoxanthin, antheraxanthin, neoxanthin, zeaxanthin, phytoen, and phytofluen.

In the course of the present invention the phrase "carotenes and carotenoids" shall also cover typical products which are obtained by the cleavage of the cited compounds, like e.g. 3,7-Dimethyl-9-(2,6,6-trimethyl-1-cyclohexenyl)-2,4,6,8-nonatetraen-1-ol (Retinol, Vitamin A1) and 3,7-Dimethyl-9-(2,6,6-trimethyl-1-cyclohexenyl)-2,4,6,8-nonatetraenal (Retinal, Vitamin Al-Aldehyde).

### Algae

The process of the present invention is directed to algae having the ability for the biosynthesis of carotenes and carotenoids. Without limiting the invention typical examples are *Chlamydomonas reinhardtii, Chlorella sp, Chlorella fusca, Chlorella salina, Chlorella sorokiniana, Chlorella stigmatofora, Chlorella vulgaris, Chlorella autotrophica, Chlorella luteoviridis, Chlorella protothecoides, Chlorococcum sp, Chlorococcum citriforme, Cyanidium caldarium, Euglena sp., Euglena gracilis, Monoraphidium braunii, Muriellopsis sp. , Nannochloropsis gaditana, Nannochloris sp, Nannochloris maculata, Nannochloris oculata, Nannochloropsis salina, Navicula pelliculosa, Neospongiococcum gelatinosum, Scenedesmus vacuolatus, Porphyridium purpureum, Dunaliella sp., Dunaliella tericiolecta, Micromonas pusilla, Neospongiococcum excentricum, Porphyridium cruentum, Pynococcus provasolli, Scenedesmus armatus, Scenedesmus obliquus, Scenedesmus quadricauda, Anacystis nidulans, Oscillatoria nubescens, Aphanocapsa sp., Gymnodinium sp., Microcystis aeruginosa, Athrospira sp., Phormidium laminosum, Phormidium persicium, Phormidium luridum,* and *Phormidium faveolarum*.

Preferred examples are *Dunaliella salina, Haematococcus pluvialis, Haematococcus lacustris, Murielopsis sphaerica,* and *Chlorella zofingiensis.*

*Dunaliella* and *Haematococcus,* e.g., represent typical examples of green micro-algae which under certain environmental conditions are able to accumulate a large amount of carotenes or carotenoids, like beta-carotene or astaxanthin. The micro-algae *Haematococcus* shows a unique life cycle, comprising several stages comprising green flagellates palmelloids, cysts, zygotes, red flagellates and gametes, but in principle one can distinguish two stages also for *Dunaliella* and *Haematococcus:* a green, motile, vegetative stage in which the cells continuously divide and synthesize chlorophyll, and a red, non-motile resting stage (cyst) in which cell division stops, chlorophyll content remains constant and carotene/carotenoid content and cellular dry weight continuously increase. The optimal environmental and nutritional conditions required for these two stages are quite different. For vegetative growth, full nutrient medium, moderate light intensity and adequate temperature and pH value are essential. For the resting stage, however, no nutrient (except carbon) is required and - usually - a higher light intensity (e.g., sunlight) is necessary for faster active accumulation. Due to such a discrepancy, the two stages are usually separated into different cultivating systems with an according different medium. The growth strategies for each stage are also different. In the Green Phase, the optimal conditions for cell division are maintained to achieve the maximum cell number.

In the Red Phase, the optimal inductive conditions must be provided for carotene/carotenoid accumulation. A preferred embodiment of the present invention is to obtain astaxanthin from *Haematococcus* strains. For the purposes of the process of the invention, any number of strains of *Haematococcus* may be employed, for example, strains such as those shown in Table I below.

**Table I**

| **Haematococcus Strains** | |
|---|---|
| **Haematococcus Strains and Species** | **Source** |
| *H. pluvialis flotow* CCAG | CCAG, Göttingen Germany) |
| *H. pluvialis flotow* NIES | NIES, Tsukaba, Japan |
| *H. pluvialisflotow* ETTL 1958/3 | ETTL, Czech Republic |
| *H. pluvialis flotow* TAKAOOVA 1983/1 | ETIL, Czech Republic |
| *H pluvialis flotow* 1844 em. Willie K-0084 | SCCAP, Denmark |
| *H. lacustris* UTEX 16 | CCAT, USA |
| *H. lacustris* ATCC 30453 | ATCC, Maryland (U.S.A) |
| *H. lacustris* CCAP 34/8 | CCAP, Scotland |
| *H. lacustris* NIES 144 | NIES, Tsukaba, Japan |
| *H. lacustris* 192.80 | CCAG, Göttingen, Germany |
| *H. lacustris* SAG 34-1b | CCAG, Göttingen, Germany |
| *H. Droechkensis* CCAP 43/G | CCAP, Great Britain |
| *H. Cognis* 2004 | Cognis GmbH |

The above strains are well documented in the art. The preferred strain in accordance with the invention is the strain exemplified herein below, *H. pluvialis Flo.* (K-0084), which has amongst its characteristics a very low tendency to clump, making it particularly useful for large-scale cultures. It is to be understood that any other strain of *Haematococcus* which may be effectively subjected to the processes according to the invention would also be employable in accordance with the invention.

### General conditions for the harvesting of algae cells

Many culture conditions and culture media are known for small-scale stock cultures and large-scale cultures of algae cells. However, for the purposes of the present invention, it has been found that the optimal culture conditions and media are actually the relatively simple culture conditions and media described herein below, these therefore being the preferred ones in accordance with the invention.

Light intensity both in the Green and in the Red Phases could be anywhere in the range from about 30 to 200, preferably 50 to 150, and more preferably 80 to 120 µE*m⁻²*s⁻¹.

It has been found that the cells can be cultivated both in the Green and in the Red Phase at a temperature in the range of 5 to 35, more particularly between 18 and 28 °C. The upper limit for the temperature in the Red Phase could in fact reach 35 °C, while the lower limit in this stage could be as low as 5 °C. The best results were obtained when the upper limit is maintained below 32 °C, and the lower limit at 15 °C.

In a further preferred embodiment of the invention the algae are grown mixotrophically (with additional nutrients to enhance cell growth), more particularly the algae are grown mixotrophically in the Green Phase. More preferably, the algae are grown mixotrophically in the Green Phase and the stress takes place preferably in absence of the nutrients of the mixotrophic Green Phase.

### Chemical stress factors

In contrast to the prejudice that high oxygen concentrations in the cultivation of algae have a negative influence and may cause photo-inhibition for the production of actives, it has surprisingly been found that chemical stress factors, preferably substances releasing active oxygen-species like singlet oxygen, radical oxygen or peroxy compounds, and more preferably hydrogen peroxide stimulate the production of carotenes and carotenoids in algae. As a matter of fact, the chemical stressing of the cells has been found to be more efficient compared to other stress factors, e.g., enhancement of light intensity or deprivation of nutrients (although additional enhancement of light intensity is not a disadvantage, but the add-on increase of productivity is usually overcompensated by the economic factors). Usually, said chemical stress factors are added in an amount of 0.05 to 5, preferably 0.1 to 1 mmol per litre of culture suspension.

Preferably, these stress factors are added to the cultivated cells after the Green Phase has been finished. More particularly the stressing of the cultivated cell can take place in one step or repeatedly over the whole period of the Red Phase. Although the biosynthesis has not been clarified in detail, it appears that the addition of chemical stress factors induces enzymes of the carotenoid biosynthetic pathway which are responsible for the production of anti-oxidants in order to protect the plant cells.

It was observed that under the above growth and stress conditions the rate of production of algae cells, in particular *Haematococcus* cells, was about 0.35 to 0. 5 g/l d, and the cells contained about 3 to 4 % b.w. carotenes or carotenoids, particular astaxanthin. Usually, the Red Phase cultivation procedure was performed for about four to six days before harvesting and drying of the cells. The above productivity of carotenes or carotenoids, particularly of astaxanthin, has not been achieved in any previously described large-scale process, since it provides a number of advantages over the previously described procedures: it is highly efficient for algae cell biomass increase as well as for the induction and accumulation of carotenes and carotenoids in these cells. The most interesting goal for the economic production of these actives is productivity, which means produced mass of carotenes or carotenoids per reactor volume and time. This figure has the biggest influence on the production costs and on the success on the market if this process is compared with other processes, e.g., for the production of astaxanthin like chemical synthesis or the production by the yeast *Xanthophyllomyces dendrorhous* (formerly known as *Phaffia rhodozyma*).

### Detailed process and cultivating conditions

While many apparatuses, systems and conditions have been described for the growth of algae cells, none of these were able to address the optimisation of the two phases of cell growth simply, efficiently and inexpensively. Accordingly, the preferred system, apparatus and conditions of the present invention are those exemplified herein below.

### Green Phase

In a preferred embodiment of the present invention, the algae strains are grown in a Modified BG-11 growth medium containing:
(i) 0. 32 g/l K₂HPO₄·3H₂O;
(ii) 0.2 g/l MgSO₄·7H₂O; and
(iii) 0.1 g/l Na₂CO₃,
in addition to the other mineral constituents contained in the standard BG-11 growth medium.

The above addition of phosphate, magnesium and carbonate has been presently shown to provide optimal conditions for the green- stage growth of the cells. The non-modified BG-11 medium cannot support long-term cultures or dense cultures due to its having less of these additives, such cultures in non-modified BG-11 often entering the Red Phase of growth earlier than desired, i.e., before the optimal amount of "green" cells has been obtained. Basal medium is usually mixed with the Modified BG-11 medium for optimal growth of the algae strain in the initial stage only of growing the cells on agar plates or slants. The Basal Medium used was the Standard Basal Medium as described by Kobayashi et al. (1991).

The Phase I growth procedure has the following steps:
(a) Cultivation of algae strains on solid agar slants or plates: An axenic strain is grown and maintained in solid agar slants or plates containing a mixture of Modified BG-11 medium and Basal Medium (1:1 v/v) under conditions of light intensity ranging between 30 to 40 µE*m⁻² s⁻¹ at 20 °C.
(b) Cultivation of liquid stock cultures of the algae: Algae strains from agar plates or slants of (a) above are inoculated into 100 ml of liquid medium (being the Modified BG-11 medium as in (a)) in 250 ml culture flasks and subsequently in larger flasks (or even glass columns) of between 500 ml to 2.5 1 in volume. The flasks are incubated in a standard gyratory incubator having a controllable light source under conditions of light intensity ranging between 50 to 70 µE*m⁻²s⁻¹ at 23°C (or when glass columns were used, agitation was by way of aeration with an air mixture containing 1.5% CO₂ v/v). The algae cultures obtained in these liquid stock cultures are cultures of the so-called "green culture" type, the algae cells being in the above- noted Green Phase of growth.

### Photo-bioreactor

In a further preferred embodiment of the present invention, the cells are cultivated in a photo-bioreactor, preferably a tubular or a panel photo-bioreactor, having the advantage of a very large surface area with respect to its volume for optimal large-scale production of algae cells in the Green Phase of growth. Usually, such bioreactors have a volume in the range from 100 to 35,000 litres, depending on the scale of production that is desired, and which contain as integral components commercially available PVC, acrylic (Plexiglas), polycarbonate or glass tubes having a diameter of about 3 to 5 cm. In operation, the culture cells in tap water, to which CO₂ is added, is circulated through the device using a pump. The green cultures from the liquid stock cultures (or inoccula) are inoculated into said photo-bioreactor, in which the algae are spurged with a mixture of 1.5% CO₂ in air. Spurging and pumping also served to prevent clumping of the cells. It should be mentioned that in a special embodiment the process can be carried out in a photo-bioreactor without collecting the algae mass between the Green and Red Phases.

The initial cell concentration in the photo-bioreactor is preferably adjusted to between about 0.1 to 0.3 * 10⁶ cells/ml at cell concentrations (dry mass) during process cultivation between 0,1 - 8 g dry biomass /1, by dilution with fresh modified BG-11 medium. The light intensity is usually kept in the range of between 70 to 90 µE*m⁻²s⁻¹ as provided by standard white cool fluorescent lamps having a 40 watt rating. It has been found advantageous to maintain the temperature in the photo-bioreactor in the range between 22- 25°C. Under these culture conditions, one can obtain a rate of algae cells, especially of *Haematococcus* cell production in the range of at least 0.7 g cells/l culture/day.

By using a glass house as an indoor cultivation room it is easy to keep temperatures below 32 °C in middle and northern Europe in summer time. In addition, the tubular photo-bioreactor and the indoor placement have the advantage of clean, long term and controlled operation, and if the photo-bioreactor is made of glass tubes instead of plastic it does not require expensive maintenance or revamping to operate for a long time. Further, the culture conditions are extremely inexpensive, the cells being grown in tap water with the addition of carbon dioxide as, essentially, the major nutrient source.

### Red Phase

In another preferred embodiment of the inventive process the cells obtained from the Green Phase cultivation procedure are used as inoccula for the photo-bioreactor. The green cells are inoculated in the form of a suspension of green cells in tap water, usually the green cells obtained from the Green Phase cultivation being re-suspended in tap water in an approximately 1 : 3 dilution resulting in biomass concentrations of 0.5 to 5 g/L dry matter. CO₂ is added to the suspension of the green cells in the bioreactor by injection a premix with air. The temperature in the bioreactor is advantageously maintained at temperatures below 32°C.

It should be noted, however, that in a number of experiments it was found that the cells from the green stage of growth could be collected, concentrated by natural sedimentation due to their tendency to sediment, and stored in a cold room (about 5°C) for up to about 2 to 3 weeks, before their inoculation into the photo-bioreactor of the Red Phase of growth. Cells collected and stored this way may also be depleted of their initial nutrient media, which also serves to induce stress in the cells and, hence, carotene or carotenoids production as taught in EP 0877817 B1.

It was observed that under the above Phase II growth conditions, the rate of production of algae cells was about 0.4 g/l*d, and that the algae cells contained about 4 % b.w. carotene or carotenoid. Usually, the Red Phase cultivation procedure is usually performed for about four to six days before harvesting and drying of the cells which are rich in carotene or carotenoid. In case that the cultivation takes place outdoors, such cultures, especially those grown in the summer months when ambient temperatures can reach, in some places, degrees as high as 40 to 45 °C, temperature control is crucial. As noted above, it is necessary to maintain the temperature in the bioreactor below 32 °C for optimal cultivation of the algae cells, this having been achieved by use of a water spray, whereby cold water was sprayed directly onto the bioreactor to maintain its temperature below 32 °C.

Further, in the Red Phase of growth, the algae cells require only carbon as the major nutrient source, this having been supplied, as noted above, by injection of CO₂. This injection of CO₂ may be performed during the day and during the night. However, the CO₂ is essential only during the day and thus a further saving of CO₂ may be achieved by stopping the injection during the night.

### Purification

In order to obtain a carotene- or carotenoids-enriched product from the induced "red" algae cells, many procedures have been described, for example, the procedures in WO 89/006910**.** While these procedures may be employed in accordance with the present invention, the preferred procedure is that of centrifugation or filtration under vacuum to concentrate the cells and drying of the concentrated cells. The dried cell mass is then preferably stored at low temperatures (e.g., - 20°C or even lower) under oxygen-free conditions, e.g., by vacuum packing, or preferably, by introduction into plastic bags or the like together with nitrogen (N₂) to remove the oxygen.

In another preferred embodiment of the invention, the process comprises the following additional steps:
(c) Harvesting the cells cultivated in step (b) by collecting said cells to form a concentrated suspension,
(d) Adding antioxidants and emulsifiers to said suspension, disrupting the collected cells and drying them to obtain a carotene- or carotenoid-enriched product.

Generally, the collection and concentration of said cells is carried out by centrifugation or filtration under vacuum, and wherein drying of said cells is done by lyophilisation, combined drying and grinding (air-vortex-mill), or spray drying.

More particularly, following the Red Phase, each cultivation cycle optimally being about four to six days, the following harvesting procedure is performed based on the fact that algae cells, especially red cells, readily sediment once collected from the photo-bioreactor. Thus, the red cell biomass from the photo-bioreactor is collected into a standard large volume funnel, e.g. an Imhoff funnel, and left to stand for a few hours (about 3-5 hours) to facilitate sedimentation of the red cells. It was found that approximately 30 % b.w. of the total volume of biomass from the bioreactor represented the red cell sediment while the remaining approx. 70 % b.w. of the total collected volume represented the tap water used in the Red Phase cultivation. This tap water can thus be easily collected and used for a new bioreactor inoculation and Red Phase cultivation procedure (i.e., the originally used tap water is almost completely recyclable). The above precipitated and concentrated red cell culture is then collected from the funnel and subjected to centrifugation or vacuum filtration to further concentrate the red cells. Routinely, a biomass yield of about 40 % b.w. solids (paste of red cells) is obtained following the centrifugation step, or about 30 % b.w. solids following vacuum filtration. Here, too, the approximately 60 % b.w. of the total volume subjected to centrifugation, or approximately 70 % b.w. of the total volume subjected to vacuum filtration, being the supernatant volume, could also be collected and used for another round of the Red Phase cultivation procedure, this supernatant being primarily the original tap water used in the Red Phase procedure.

The concentrated red cell slurry obtained from the above centrifugation step is then homogenized and stabilised by adding anti-oxidants and then dried, preferably by lyophilisation, although spray drying also proved to be effective. Such antioxidants are selected from the group consisting of ethoxyquin, butylated hydroxyanisole, butylated hydroxytoluene (BHT), tocopherols, di-tert-butyl-paracresol and propyl gallate. The preferred antioxidant, however, is a natural tocopherol product containing 30 % b.w. of alpha tocopherol to have at least a real natural product. Usually, the amount of antioxidant added in the grinding procedure will range from about 0.05 to 5 % (w/w) of the amount of dry powder. The powders are packed into a plastic bag pre-filled with nitrogen gas to remove oxygen (which causes pigment oxidation, i.e. degradation of the active) and are then stored at -20 °C prior, e.g., to processing to prepare the food additive for fish coloration.

The final stage of producing a carotene- or carotenoids-enriched product in the form of small particles easily digested by fish may also be carried out in a number of ways as previously described in the art. The preferred procedure is the one exemplified herein below, which involves the use of a standard ball mill in which the biomass slurry is disintegrated as a suspension in water in the presence of any suitable anti-oxidant to prevent oxidation of the astaxanthin-enriched product. After drying, this yields a powder-like product of small particle size, particularly useful as an additive to fish meal, these small astaxanthin-rich particles being easily digested.

The powder as obtained from the above procedure may then be utilized directly or in admixture with other ingredients as an additive to fish meal for the sake of coloration or in food applications like dietary supplements. In another process, the carotenes or carotenoids can be concentrated by an extraction process to use them for formulation of food supplement or pharmaceutical products.

### Examples

### Selection of Haematococcus strains

From the great variety of *Haematococcus* strains the following were used:
● **ATCC 30453,** American Type Culture Collection, Rockville, Maryland (Synonym *Haematococcus* lacustris)
● **CCAP 34/8,** Culture Collection of Algae and Protozoa, Scotland
● **Nies 144,** Culture Collection Japan
● **192.80,** Culture Collection, Göttingen, (Synonym *Haematococcus* lacustris)
● **SAG 34-1b,** Culture Collection, Göttingen

In addition a new *Haematococcus* strain was used resulting from mutagenesis and cell sorting, named **Cognis2004.** The procedure for cultivating the astaxanthin producing strains of *Haematococcus* in accordance with the present invention consists of two phases, green and red as described below.

### Green Phase cultivation of Haematococcus strains

For the Green Phase cultivation a "Modified OHM-N-BG 11 medium" was used containing:
(i) 0.32 g/l K₂HPO₄ *3H₂O
(ii) 0.2 g/l MgSO₄ * 7H2O and
(iii) 0.1 g/l Na₂CO₃,
in addition to the other mineral constituents contained in the standard OHM growth medium as, for example, described in US 6,02,2701**,** which is hereby incorporated by reference.

Cultivation of liquid stock cultures of *Haematococcus* cells into 100 ml of liquid medium as in 250 ml culture flasks and subsequently in larger flasks of between 500 ml-2.0 1 in volume. The flasks were incubated in a standard gyratory incubator having a controllable light source under conditions of light intensity ranging between 30 - 60 µE/m²/s at 25 °C. The cultures of *Haematococcus* obtained in these liquid stock cultures are cultures of the so-called "green culture" type, which means planospores and a high amount of small cells.

### Example 1

### Production of large-scale cultures of Haematococcus in a tubular photo-bioreactor

### I.

### The Green Phase cultivation procedure

The green cultures from the liquid stock cultures of above were inoculated into a photo-bioreactor, in which the algae are spurged with a mixture of 1.5% CO₂ in air and pumped through the tubular photo-receiver part of the bioreactor. Pumping and spurging also serve to prevent clumping of the cells. The photo-bioreactor used for these large-scale cultures was a glass tubular type developed by IGV Potsdam. In this photo-bioreactor, the initial *Haematococcus* cell concentration was adjusted to between about 0.1 to 0. 5 g/l, by dilution with fresh modified BG-11 medium. The light intensity was kept in the range of between 120 - 180 µE*m⁻²s⁻¹ as provided by standard white cool fluorescent lamps having a 40 watt rating. The temperature in the photo-bioreactor was maintained in the range of 25 °C. Under these culture conditions, it was possible to obtain a rate of *Haematococcus* cell production in the range between 0.2 -1.0g/l day. Usually, the Green Phase cultivation procedure was performed as repeated-batch fermentation with partial harvesting of green cells every second day of cultivation.

### II.

### The Red Phase cultivation procedure

The cultivation device used was a standard tubular photo-bioreactor available with volumes ranging from 30 to 35,000 litres. In operation, the culture (*Haematococcus* cells in tap water, to which CO₂ is added) is circulated through the device using a pump. For the cultivation of the *Haematococcus* cells in their "red stage" of growth, the green cells were used as inoccula for the above tubular photo-bioreactor. The tubular photo-bioreactor was placed in a glass house. The green cells were inoculated in the form of a suspension of green cells in tap water, and the green cells obtained from Green Phase cultivation being re-suspended in tap water in an approximately 1 : 3 dilution. CO₂ was added to the suspension of the green cells in the tubular photo-bioreactor. The temperature in the tubular photo-bioreactor was maintained at temperatures below 32 °C.

In the following, a non-recurring addition of 1mmol hydrogen peroxide to the cultivation broth and fermentation for five days at light intensities below 200 µE*m⁻²s⁻¹ took place and resulted in biomass containing between 3 and 3.5% of astaxanthin in the case of *Haematococcus.* In comparison, cultivation without addition of a chemical stressor took at minimum 7 days and needed light intensities between 800 and 1,500 µE*m⁻²s⁻¹ to achieve a carotenoid content of 3.5% based on dry matter.

The Red Phase cultivation procedure was performed for four to five days before harvesting and drying of the cells. The rate astaxanthin production reached a level of 0.42 g/l d, which means that the *Haematococcus* cells contained about 3 % b.w. astaxanthin.

### III.

### Harvesting of Haematococcus

Following the Red Phase cultivation of *Haematococcus* cells, each cultivation cycle optimally being about three to six days, the following harvesting procedure was performed, based on the fact that *Haematococcus* cells (especially red cells), readily sediment once collected from the tubular photo-bioreactor. Thus, the red cell biomass from the tubular photo-bioreactor was collected into a standard large volume vessel from which it can be further concentrated by means of centrifugation or filtration. The separated water phase could thus be easily collected and re-used for a new tubular bioreactor inoculation and cultivation procedure. A biomass concentration of about 25 % b.w. solids was obtained following the centrifugation step. The concentrated suspension of red cells obtained from the above centrifugation step was then disintegrated in a ball mill, stabilized by addition of 0.15 (w/w) natural tocopherol and dried, preferably by spray drying. The resulting dried powder was packed into aluminium bags pre-filled with nitrogen gas to remove oxygen and then stored.

## Claims

1. A process for the production of carotenes or carotenoids from algae or cyanobacteria comprising
(a) cultivating algae or cyanobacteria having the ability for the biosynthesis of carotenes or carotenoids under conditions suitable for optimal vegetable growth of said cells in the so-called "Green Phase", and
(b) collecting the cells thus obtained and cultivating them at temperatures in the range of 5 to 35 °C and light intensities of 30 to 200 µE*m⁻²*s⁻¹ in the so-called "Red Phase",
**characterised in that** the light intensity in step (b) is not increased compared to step (a) and that the cultivated cells are subjected to chemical stress by adding a substance releasing active oxygen species for the induction of the carotene or carotenoid synthesis in one step or repeatedly over the whole period of the Red Phase.

2. A process according to claim 1, **characterised in that** algae are cultivated which have the ability for the biosynthesis of carotenes or carotenoids selected from the group consisting of beta-carotene, capsanthin, bixin, capsorubin, astaxanthin, lutein, lycopene, violaxanthin, cryptoxanthin, antheraxanthin, neoxanthin, canthaxanthin, zeaxanthin, phytoen, phytofluen, retinol and retinal.

3. A process according to claim 1 and/or 2, **characterised in that** algae are cultivated which are selected from the group consisting of *Dunaliella sp, Dunaliella salina, Dunaliella tertiolecta, Haematococcus sp, Haematococcus pluvialis, Haematococcus lacustris, Murielopsis sp, Anabaena spaerica, Chlorella sp, Chlorella zofingiensis, Chlorococcum sp, Neospongiuococcum sp, Coelastrum sp,* and *Scenedesmus sp.*

4. A process according to any of the claims 1 to 3, **characterised in that** the cells are subjected to the same light intensities both in the Green and in the Red Phase in the range of about 30 to 200 µE*m⁻²*s⁻¹.

5. A process according to any of the claims 1 to 4, **characterised in that** the cells are cultivated both in the Green and in the Red Phase at a temperature in the range of 5 to 35 °C.

6. A process according to any of the claims 1 to 5, **characterised in that** the algae are grown mixotrophically.

7. A process according to claim 6, **characterised in that** the algae are grown mixotrophically in the Green Phase.

8. A process according to claims 6 and/or 7, **characterised in that** the algae are grown mixotrophically in the Green Phase and the stress takes place in the absence of the nutrients of the mixotrophic Green Phase.

9. A process according to any of the claims 1 to 8, **characterised in that** the cells are subjected to chemical stress by addition of substances releasing active oxygen-species selected from singlet oxygen, radical oxygen or peroxy compounds.

10. A process according to any of the claims 1 to 9, **characterised in that** the cells are subjected to chemical stress by addition of hydrogen peroxide.

11. A process according to claims 9 and/or 10, **characterised in that** said chemical stress factors are added in an amount of 0.05 to 5 mmol per litre of culture suspension.

12. A process according to any of the claims 1 to 11, **characterised in that** said cells are inoculated into a growth solution consisting of tap water to which carbon dioxide is added.

13. A process according to any of the claims 1 to 12, **characterised in that** said cells are cultivated in a photo-bioreactor.

14. A process according to claim 13, **characterised in that** said photo-bioreactor is a tubular or panel bioreactor.

15. A process according to any of the claims 1 to 14, **characterised in that** the initial concentration of cells used for the cultivation is about 0.1 to 0.3 * 10⁶ cells/ml at cell concentrations (dry mass) during process cultivation between 0,1 - 8 g dry biomass /1.

16. A process according to any of the claims 1 to 15, **characterised in that** it comprises the following additional steps:
(c) harvesting the cells cultivated in step (b) by collecting said cells to form a concentrated suspension,
(d) adding antioxidants and/or emulsifiers to said suspension, disrupting the collected cells and drying them to obtain a carotene- or carotenoids-enriched product.

17. A process according to claim 16, **characterised in that** the collection and concentration of said cells is carried out by sedimentation followed by centrifugation or filtration under vacuum, and wherein the drying of said cells is done by lyophilisation, combined drying and grinding or spray drying.

18. A process according to any of the claims 16 and/or 17, **characterised in that** the antioxidants are selected from the group consisting of ethoxyquin, butylated hydroxyanisole, butylated hydroxytoluene (BHT), tocopherols, di-tert-butyl-paracresol and propyl gallate.

## Patentansprüche

1. Verfahren zur Herstellung von Carotinen oder Carotinoiden aus Algen oder Cyanobakterien umfassend:
(a) Kultivierung von Algen oder Cyanobakterien mit der Fähigkeit zur Biosynthese von Carotinen oder Carotinoiden unter optimalen pflanzlichen Wachstumsbedingungen der Zellen in der so genannten "Grünen Phase",
(b) Einsammeln und Kultivieren der so erhaltenen Zellen bei Temperaturen im Bereich von 5 bis 35 °C und einer Lichtintensität von 30 bis 200 µE*m⁻²*s⁻¹ in der so genannten "Roten Phase",
**dadurch gekennzeichnet, dass** die Lichtintensität im Schritt (b) im Vergleich zum Schritt (a) nicht erhöht wird und die kultivierten Zellen chemischem Stress ausgesetzt werden, indem man ihnen zur Einleitung der Carotin- oder Carotinoid-Synthese einmalig oder wiederholt über den Verlauf der gesamten Roten Phase Substanzen zusetzt, die aktive Sauerstoffspezies freisetzen.

2. Verfahren nach Anspruch 1, **dadurch gekennzeichnet dass** Algen mit der Fähigkeit zur Biosynthese von solchen Carotinen oder Carotinoiden kultiviert werden, die ausgewählt sind aus der Gruppe, die gebildet wird von beta-Carotin, Capsantin, Bixin, Capsorubin, Astaxantin, Lutein, Lycopen, Violaxantin, Cryptoxantin, Antheraxantin, Neoxantin, Canthaxantin, Zeaxantin, Phytoen, Phytofluen, Retinol und Retinal.

3. Verfahren nach den Ansprüchen 1 und/oder 2, **dadurch gekennzeichnet, dass** Algen kultiviert werden, die ausgewählt sind aus der Gruppe, die gebildet wird von *Dunaliella sp, Dunaliella salina, Dunaliella tertiolecta, Haematococcus sp, Haematococcus pluvialis, Haematococcus lacustris, Murielopsis sp, Anabaena spaerica, Chlorella sp, Chlorella zofingiensis, Chlorococcum sp, Neospongiuococcum sp, Coelastrum sp,* und *Scenedesmus sp.*

4. Verfahren nach jedem der Ansprüche 1 bis 3, **dadurch gekennzeichnet, dass** die Zellen gleichen Lichtintensitäten in der Grünen und in der Roten Phase von etwa 30 bis 200 µE*m⁻²*s⁻¹ ausgesetzt werden.

5. Verfahren nach jedem der Ansprüche 1 bis 4, **dadurch gekennzeichnet, dass** die Zellen sowohl in der Grünen als auch der Roten Phase bei Temperaturen im Bereich von 5 bis 35 °C kultiviert werden.

6. Verfahren nach jedem der Ansprüche 1 bis 5, **dadurch gekennzeichnet, dass** die Algen unter mixotrophen Bedingungen wachsen.

7. Verfahren nach Anspruch 6, **dadurch gekennzeichnet, dass** die Algen in der Grünen Phase unter mixotrophen Bedingungen wachsen.

8. Verfahren nach den Ansprüchen 6 und/oder 7, **dadurch gekennzeichnet, dass** die Algen in der Grünen Phase unter mixotrophen Bedingungen wachsen und die Stressung in Abwesenheit von Nährstoffen in der mixotrophen Grünen Phase stattfindet.

9. Verfahren nach jedem der Ansprüche 1 bis 8, **dadurch gekennzeichnet, dass** die Zellen chemischem Stress durch Zugabe von Stoffen ausgesetzt werden, die freie Sauerstoffspezies freisetzen, welche ausgewählt sind aus Singulett-Sauerstoff, Sauerstoffradikalen und Peroxyverbindungen.

10. Verfahren nach jedem der Ansprüche 1 bis 10, **dadurch gekennzeichnet, dass** die Zellen chemischem Stress durch Zugabe von Wasserstoffperoxid ausgesetzt werden.

11. Verfahren nach den Ansprüchen 9 und/oder 10, **dadurch gekennzeichnet, dass** besagte chemische Stressfaktoren in Mengen von 0,05 bis 5 mMol/l bezogen auf die Kultivierungssuspension zugegeben werden.

12. Verfahren nach jedem der Ansprüche 1 bis 11, **dadurch gekennzeichnet, dass** die Zellen in eine Wachstumslösung bestehend aus Leitungswasser, welchem Kohlendioxid zugesetzt wurde, eingesetzt werden.

13. Verfahren nach jedem der Ansprüche 1 bis 12, **dadurch gekennzeichnet, dass** die Zellen in einem Photo-Bioreaktor kultiviert werden.

14. Verfahren nach Anspruch 13, **dadurch gekennzeichnet, dass** der Photo-Bioreaktor ein Röhren- oder Scheibenbioreaktor ist.

15. Verfahren nach jedem der Ansprüche 1 bis 14, **dadurch gekennzeichnet, dass** die Startkonzentration der Zellen für die Kultivierung etwa 0,1 bis 0,3*10⁶ Zellen/ml bei einer Zellkonzentration (Trockenmasse) während des Kultivierungsprozesses von 0,1 bis 8 g trockener Biomasse/l beträgt.

16. Verfahren nach jedem der Ansprüche 1 bis 15, **dadurch gekennzeichnet**, das es die weiteren zusätzlichen Schritte umfasst:
(c) Ernten der in Schritt (b) kultivierten Zellen durch Einsammeln der Zellen unter Gewinnung einer konzentrierten Suspension;
(d) Zugabe von Antioxidantien und/oder Emulgatoren zu der Suspension, Aufbrechen der gesammelten Zellen und Trocknen unter Gewinnung eines carotin- oder carotinoidreichen Produktes.

17. Verfahren nach Anspruch 16, **dadurch gekennzeichnet, dass** das Einsammeln und Aufkonzentrieren besagter Zellen durch Sedimentation durchgeführt wird, welcher sich eine Zentrifugierung oder Vakuumfiltration anschließt, und wobei die Trocknung der Zellen durch Gefriertrocknung, Trocknungsvermahlung oder Sprühtrocknung erfolgt.

18. Verfahren nach den Ansprüchen 16 und/oder 17, **dadurch gekennzeichnet, dass** die Antioxidantien ausgewählt sind aus der Gruppe bestehend aus Ethoxyquin, butyliertem Hydroxyanisol, butyliertem Hydroxytoluol (BHT), Tocopherolen, Di.tert.butyl-Paracresol und Propylgallat.

## Revendications

1. Procédé de production de carotènes ou de caroténoïdes à partir d'algues ou de cyanobactéries comprenant
(a) la culture d'algues ou de cyanobactéries ayant la capacité de biosynthèse de carotènes ou de caroténoïdes dans des conditions appropriées pour une croissance végétale optimale desdites cellules dans la phase dite « verte », et
(b) la collecte des cellules ainsi obtenues et leur culture à des températures comprises dans la gamme de 5 à 35 °C et des intensités lumineuses de 30 à 200 µE.m⁻².s⁻¹ dans la phase dite « rouge »,
**caractérisé en ce que** l'intensité lumineuse à l'étape (b) n'est pas augmentée en comparaison de l'étape (a) et **en ce que** les cellules cultivées sont soumises à un stress chimique en ajoutant une substance libérant des espèces actives de l'oxygène pour l'induction de la synthèse de carotènes ou de caroténoïdes en une seule étape ou de façon répétée sur la période entière de la phase rouge.

2. Procédé selon la revendication 1, **caractérisé en ce que** sont cultivées des algues qui ont la capacité de biosynthèse de carotènes ou de caroténoïdes choisis dans le groupe constitué par le bêta-carotène, la capsanthine, la bixine, la capsorubine, l'astaxanthine, la lutéine, le lycopène, la violaxanthine, la cryptoxanthine, l'anthéraxanthine, la néoxanthine, la canthaxanthine, la zéaxanthine, le phytoène, le phytofluène, le rétinol et le rétinal.

3. Procédé selon les revendications 1 et/ou 2, **caractérisé en ce que** sont cultivées des algues qui sont choisies dans le groupe constitué par *Dunaliella sp., Dunaliella salina, Dunaliella tertiolecta, Haematococcus sp., Haematococcus pluvialis, Haematococcus lacustris, Muriellopsis sp., Anabaena sperica, Chlorella sp., Chlorella zofingiensis, Chlorococcum sp., Neospongiococcum sp., Coelastrum sp.,* et *Scenedesmus sp..*

4. Procédé selon l'une quelconque des revendications 1 à 3, **caractérisé en ce que** les cellules sont soumises aux mêmes intensités lumineuses à la fois dans la phase verte et dans la phase rouge, dans la gamme d'environ 30 à 200 uE.m⁻².s⁻¹.

5. Procédé selon l'une quelconque des revendications 1 à 4, **caractérisé en ce que** les cellules sont cultivées à la fois dans la phase verte et dans la phase rouge à une température comprise dans la gamme de 5 à 35 °C.

6. Procédé selon l'une quelconque des revendications 1 à 5, **caractérisé en ce que** les algues sont cultivées dans des conditions mixotrophes.

7. Procédé selon la revendication 6, **caractérisé en ce que** les algues sont cultivées dans des conditions mixotrophes dans la phase verte.

8. Procédé selon les revendications 6 et/ou 7, **caractérisé en ce que** les algues sont cultivées dans des conditions mixotrophes dans la phase verte et le stress survient en l'absence des nutriments de la phase verte mixotrophe.

9. Procédé selon l'une quelconque des revendications 1 à 8, **caractérisé en ce que** les cellules sont soumises à un stress chimique par addition de substances libérant des espèces actives de l'oxygène choisies parmi l'oxygène singulet, l'oxygène radicalaire et les composés peroxydiques.

10. Procédé selon l'une quelconque des revendications 1 à 9, **caractérisé en ce que** les cellules sont soumises à un stress chimique par addition de peroxyde d'hydrogène.

11. Procédé selon les revendications 9 et/ou 10, **caractérisé en ce que** lesdits facteurs de stress chimique sont ajoutés dans une quantité de 0,05 à 5 mmol par litre de suspension de culture.

12. Procédé selon l'une quelconque des revendications 1 à 11, **caractérisé en ce que** lesdites cellules sont inoculées dans une solution de croissance constituée d'eau du robinet à laquelle est ajouté du dioxyde de carbone.

13. Procédé selon l'une quelconque des revendications 1 à 12, **caractérisé en ce que** les cellules sont cultivées dans un photobioréacteur.

14. Procédé selon la revendication 13, **caractérisé en ce que** ledit photobioréacteur est un bioréacteur tubulaire ou plat.

15. Procédé selon l'une quelconque des revendications 1 à 14, **caractérisé en ce que** la concentration initiale de cellules utilisée pour la culture est d'environ 0,1 à 0,3.10⁶ cellules/ml pour des concentrations de cellules (masse sèche) pendant la culture du procédé comprises entre 0,1 et 8 g de biomasse sèche/l.

16. Procédé selon l'une quelconque des revendications 1 à 15, **caractérisé en ce qu'**il comprend les étapes supplémentaires suivantes :
(c) récolter les cellules cultivées à l'étape (b) en collectant lesdites cellules pour former une suspension concentrée,
(d) ajouter des antioxydants et/ou des émulsifiants à ladite suspension, dissocier les cellules collectées et les sécher pour obtenir un produit enrichi en carotènes ou en caroténoïdes.

17. Procédé selon la revendication 16, **caractérisé en ce que** la collecte et la concentration desdites cellules sont réalisées par sédimentation suivie d'une centrifugation ou d'une filtration sous vide, et dans lequel le séchage desdites cellules est effectué par lyophilisation, séchage et broyage combinés ou séchage par atomisation.

18. Procédé selon l'une quelconque des revendications 16 et 17, **caractérisé en ce que** les antioxydants sont choisis dans le groupe constitué par l'éthoxyquine, l'hydroxyanisole butylé, l'hydroxytoluène butylé (BHT), les tocophérols, le di-tert-butyl-paracrésol et le gallate de propyle.
